# FASCICULE DE BREVET EUROPEEN

(11) **EP 2 675 825 B1**
(45) Date de publication et mention de la délivrance du brevet: **29.04.2015**
(21) Numéro de dépôt: 12709676.6
(22) Date de dépôt: 17.02.2012
(51) Int. Cl.: C07K 7/06

(54) **NOUVEAUX PEPTIDES ACTIVATEURS DE LA SYNTHESE DES PROTEINES DE LA MATRICE EXTRACELLULAIRE ET COMPOSITIONS COSMETIQUES LES COMPRENANT**
NEUE AKTIVATORPEPTIDE ZUR SYNTHESE EXTRAZELLULÄRER MATRIXPROTEINE UND KOSMETISCHE ZUSAMMENSETZUNGEN DAMIT
NOVEL EXTRACELLULAR MATRIX PROTEIN SYNTHESIS ACTIVATING PEPTIDES AND COSMETIC COMPOSITIONS CONTAINING THEM

(30) Priorité: 18.02.2011 FR 1100497
(43) Date de publication de la demande: 25.12.2013
(73) Titulaire: ISP Investments Inc., Wilmington, DE 19805 (US)
(72) Inventeur: DAL FARRA, Claude, 12446 Kerhonkson, New York (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2012/000064
(87) Numéro de publication internationale: WO 2012/140331

(56) Documents cités:
- WO-A2-2007/087131
- FR-A1- 2 940 971
- US-A1- 2007 054 298

### SEQUENCE LISTING

<110> ISP Investments INC.
<120> NOUVEAUX PEPTIDES ACTIVATEURS DE LA SYNTHESE DES PROTEINES DE LA MATRICE EXTRACELLULAIRE ET COMPOSITIONS COSMETIQUES LES COMPRENANT
<130> Bv PCT 11-150
<150> FR1100497
   <151> 2011-02-18
<160> 6
<170> PatentIn version 3.5
<210> 1
   <211> 5
   <212> PRT
   <213> Synthetic peptide
<220>
   <221> MOD_RES
   <222> (5)..(5)
   <223> AMIDATION
<400> 1
   Ala Pro Ala Gly Pro
   1               5
<210> 2
   <211> 5
   <212> PRT
   <213> Synthetic peptide
<400> 2
   Val Pro Ala Gly Pro
   1               5
<210> 3
   <211> 5
   <212> PRT
   <213> Synthetic peptide
<220>
   <221> MOD_RES
   <222> (5) .. (5)
   <223> AMIDATION
<400> 3
   Val Pro Gly Gly Pro
   1 5
<210> 4
   <211> 5
   <212> PRT
   <213> Synthetic peptide
<400> 4
   Gly Pro Ala Gly Pro
   1 5
<210> 5
   <211> 5
   <212> PRT
   <213> Synthetic peptide
<220>
   <221> MOD_RES
   <222> (5) .. (5)
   <223> AMIDATION
<400> 5
   Gly Pro Ala Gly Pro
   1 5
<210> 6
   <211> 9
   <212> PRT
   <213> Synthetic peptide
<220>
   <221> MOD_RES
   <222> (9) .. (9)
   <223> AMIDATION
<400> 6
   Lys Gly Ala Pro Gly Gly Pro Asn Tyr
   1 5

## Description

### Domaine de l'invention

La présente invention se situe dans le domaine des agents actifs cosmétiques ainsi qu'aux compositions en comportant.

La présente invention a pour objet de fournir de nouveaux peptides activateurs de l'expression des protéines de la matrice extracellulaire.

L'invention se rapporte également à des compositions cosmétiques comprenant de tels peptides, en tant qu'agents actifs, dans un milieu physiologiquement adapté.

L'invention concerne encore l'utilisation de telles compositions pour augmenter l'expression des protéines de la matrice extracellulaire, retarder l'apparition et/ou lutter contre les signes cutanés du vieillissement, et en particulier les rides, le relâchement et la perte de volume cutané et la déshydratation de la peau.

### Arrière-plan de l'invention

La peau est un organe de recouvrement composé de plusieurs couches (derme, jonction dermo-épidermique, épiderme). Le derme est le tissu de soutien de la peau et se compose d'eau, de fibres d'élastine et de fibres de collagène (70 % des fibres dermiques), enveloppées dans une matrice interstitielle de protéoglycanes. Les fibroblastes sont la principale composante cellulaire du derme et sont à l'origine de la synthèse des fibres de collagène et des fibres d'élastine.

Les glycosaminoglycanes assurent la structuration des fibrilles de collagène et d'élastine et le stockage de l'eau, grâce à leur exceptionnelle hygroscopie. L'acide hyaluronique est le glycosaminoglycane le plus abondant de la peau, et le constituant majeur du derme mais est également présent autour des kératinocytes dans l'épiderme. Les complexes de glycosaminoglycanes et de collagène sont des acteurs majeurs de la souplesse et de la fermeté de la peau.

La peau, comme tous les autres organes, est soumise au processus physiologique complexe du vieillissement. On distingue le vieillissement intrinsèque ou chronologique et le vieillissement extrinsèque. Le vieillissement intrinsèque est la conséquence d'une sénescence génétiquement programmée et d'altérations biochimiques dues à des facteurs endogènes. Au niveau de la peau, il se caractérise par un ralentissement de la régénération des cellules et des matrices extracellulaires, aboutissant à une atrophie dermique et épidermique, une sécheresse, une réduction de l'élasticité, et l'apparition de ridules et de rides.

Le vieillissement extrinsèque, quant à lui, est dû aux agressions environnementales telles que la pollution, le soleil, les maladies, les habitudes de vie, etc. Il se superpose au vieillissement intrinsèque au niveau des zones chroniquement exposées à ces agressions; on parle alors de photovieillissement. Les principales altérations liées au photovieillissement comprennent : l'apparition de tâches pigmentaires ainsi qu'une diminution, une désorganisation des fibres de collagène provoquant l'apparition de rides.

Les recherches pour identifier des agents actifs capables de lutter contre le vieillissement cutané ont abouti à la mise sur le marché de nombreux agents actifs plus ou moins efficaces. Toutefois, il reste toujours d'actualité d'identifier de nouveaux composés capables de retarder l'apparition ou de lutter plus efficacement contre les signes du vieillissement cutané. Le problème plus particulièrement visé par l'invention est d'identifier de nouveaux agents actifs capables de lutter contre les principaux signes du vieillissement cutané siégeant au niveau de la matrice extracellulaire.

### Exposé de l'invention

Les inventeurs ont mis en évidence que des peptides de formule générale (I) suivante :

R₁-(AA)ₙ-X₁-Pro-X₂-Gly-Pro-X₃-X₄-(AA)ₚ-R₂

étaient de bons agents activateurs de la synthèse des protéines de la matrice extracellulaire. En conséquence, ces peptides sont adaptés pour lutter contre le vieillissement et le photovieillissement de la peau.
Les peptides selon l'invention se caractérisent par le fait qu'ils :
- augmentent l'expression des collagènes de type I et III
- augmentent l'expression de la fibronectine,
- augmentent l'expression de l'acide hyaluronique,
- réduisent les signes de sénescence des fibroblastes.

On entend par « peptide ou agent actif activateur des protéines de la matrice extracellulaire», tout peptide de formule générale (I) capable d'augmenter la quantité de collagène, de fibronectine et d'acide hyaluronique présente dans la cellule, ou sécrétée soit en augmentant la synthèse protéique par modulation directe ou indirecte de l'expression génique, soit par d'autres processus biologiques tels que la stabilisation de la protéine ou encore la stabilisation des transcrits d'ARN messager.

On entend par « signes de sénescence des fibroblastes » le profil d'expression de marqueurs biochimiques associés au phénotype de sénescence cellulaire.

On entend par peau, l'ensemble des tissus de recouvrement constituant la peau, les muqueuses et les phanères, y compris les cheveux, les cils et les sourcils.

Ainsi, l'invention a pour objet premier un peptide de formule générale (I)

R₁-(AA)ₙ-X₁-Pro-X₂-Gly-Pro-X₃-X₄-(AA)ₚ-R₂

Dans laquelle
X₁ représente la glycine ou l'alanine ou la valine,
X₂ représente la glycine ou l'alanine ou la valine,
X₃ représente l'asparagine ou la lysine ou la glutamine ou aucun acide aminé,
X₄ représente la phénylalanine ou la tyrosine ou aucun acide aminé,
AA représente un acide aminé quelconque et n et p sont des nombres entiers compris entre 0 et 2,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée, soit par un groupement de type acyle (R-CO-) dans lequel le radical R est une chaîne alkyle de C₁ à C₃₀ saturée ou insaturée et de préférence un méthyle, soit par un groupement aromatique de type benzoyle, tosyle ou benzyloxycarbonyle,
R₂ représente le groupe hydroxyle de la fonction carboxyle de l'acide aminé C-terminal, -OH, dans lequel l'atome d'hydrogène peut être substitué par une chaîne alkyle de C₁ à C₃₀, ou un groupement NH₂, NHY ou NYY, dans lequel Y représente une chaîne alkyle de C₁ à C₄,
ladite séquence de formule générale (I) étant constituée de 5 à 11 résidus d'acides aminés et pouvant être sous forme de sels, le peptide étant de séquence :
(SEQ ID n°1) : Ala-Pro-Ala-Gly-Pro-NH₂
(SEQ ID n°2) : Val-Pro-Ala-Gly-Pro
(SEQ ID n°3) : Val-Pro-Gly-Gly-Pro-NH₂
(SEQ ID n°4) : Gly-Pro-Ala-Gly-Pro
(SEQ ID n°5) : Gly-Pro-Ala-Gly-Pro-NH₂
(SEQ ID n°6) : Lys-Gly-Ala-Pro-Gly-GLy-Pro-Asn-Tyr-NH₂.

Selon un mode de réalisation particulièrement intéressant, le peptide correspond à la séquence SEQ ID n°4.

Selon un autre mode de réalisation particulièrement intéressant, le peptide correspond à la séquence SEQ ID n°5.

Les acides aminés, constituant le peptide selon l'invention et désignés sous les termes AA, peuvent être sous configuration isomérique L- et D-. De manière préférentielle, les acides aminés sont sous forme L.

Le terme « peptide » désigne un enchaînement de deux ou plusieurs acides aminés liés entre eux par des liaisons peptidiques.

Par « peptide », il faut également entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. Il est possible de protéger la fonction amine primaire de l'acide aminé N-terminal, soit par une substitution par un groupement R₁ de type acyle (R-CO-) dans lequel le radical R est une chaîne alkyle de C₁ à C₃₀ saturée ou insaturée de préférence de type méthyle, soit par une substitution par un groupement aromatique de type benzoyle, tosyle, ou benzyloxycarbonyle.

De préférence, on réalise une protection de la fonction carboxyle de l'acide aminé C-terminal, par une substitution par un groupement R₂ de type chaîne alkyle de C₁ à C₃₀, ou un groupement NH₂, NHY ou NYY avec Y représentant une chaîne alkyle de C₁ à C₄.

Le peptide selon l'invention, de séquence SEQ ID n°1 à SEQ ID n°6, peut être protégé au niveau de l'extrémité N-terminale, C-terminale ou au niveau des deux extrémités.

Le peptide de formule générale (I) selon l'invention de séquence SEQ ID n°1-6 peut être obtenu soit par synthèse chimique classique (en phase solide ou en phase homogène liquide), soit par synthèse enzymatique (Kullman et al. J. Biol. Chem., 1980, vol. 225, p. 8234) à partir d'acides aminés constitutifs.

Le peptide selon l'invention peut être d'origine naturelle ou synthétique. Préférentiellement selon l'invention, le peptide est d'origine synthétique, obtenu par synthèse chimique.

Selon l'invention, l'agent actif peut être un peptide unique ou un mélange de peptides.

Le peptide selon l'invention est avantageusement solubilisé dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants.

Par « physiologiquement adapté », on entend des solvants ou des milieux qui conviennent à une utilisation en contact avec la peau ou les phanères humains, sans risque de toxicité, d'intolérance, d'instabilité, de réponse allergique et autres effets secondaires.

Le peptide dilué est ensuite stérilisé par filtration stérile.

Après cette étape de dilution, le peptide peut être encapsulé ou inclus dans un vecteur cosmétique tels que les liposomes ou toute autre microcapsule utilisée dans le domaine de la cosmétique ou adsorbé sur des polymères organiques poudreux, des supports minéraux comme les talcs et bentonites, et plus généralement solubilisé dans, ou fixé sur, tout vecteur physiologiquement adapté.

L'invention a pour deuxième objet une composition cosmétique comprenant, dans un milieu physiologiquement adapté, un peptide de formule générale (I) de séquence SEQ ID n°1-6, en tant qu'agent actif activateur de la synthèse des protéines de la matrice extracellulaire de la peau.

Selon un mode de réalisation avantageux de l'invention, l'agent actif est présent dans les compositions à une concentration comprise entre 0,0005 et 500 mg/kg, et préférentiellement entre 0,01 et 5 mg/kg par rapport au poids total de la composition finale. Cette fourchette de concentrations représente la quantité nécessaire d'agent actif pour obtenir l'effet moléculaire recherché, à savoir, activer l'expression des collagènes de type I et III, de la fibronectine et de l'acide hyaluronique.

De manière préférée, la composition selon l'invention se présente sous une forme adaptée à l'application par voie topique comprenant un milieu physiologiquement adapté pour la peau.

On entend par « application topique », le fait d'appliquer ou d'étaler l'agent actif selon l'invention, ou une composition le contenant, sur la surface de la peau.

Ces compositions pourront notamment se présenter sous forme d'une solution aqueuse, hydro-alcoolique ou huileuse, d'une émulsion huile-dans-eau, eau-dans-huile ou émulsions multiples, de gel aqueux ou anhydre, de colloïde. Ces compositions peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'une crème, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut, par exemple, citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP, et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être présents à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Il est bien entendu que l'agent actif selon l'invention peut être utilisé seul ou bien en association avec d'autres agents actifs.

Avantageusement, les compositions utilisables selon l'invention contiennent, en outre, au moins un autre agent actif destiné à renforcer l'action de l'agent actif selon l'invention, dans le domaine de la prévention et de l'amélioration des signes cutanés du vieillissement, ou encore un autre agent actif permettant d'élargir la gamme de propriétés de la composition considérée.

On peut citer, de manière non limitative, les classes d'ingrédients suivantes : les agents régénérants, anti-âges, antirides, apaisants, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique ou la microcirculation ou la pousse des ongles ou la pousse des cheveux, les agents modulant la différenciation ou la pigmentation de l'épiderme, les agents inhibant les métallo-protéinases, ou encore les écrans ou filtres solaires.

Dans un mode plus particulier de réalisation, la composition selon l'invention comprendra, outre le peptide selon l'invention,
- au moins un composé activateur du cytochrome c et/ou,
- au moins un composé hydratant, tel qu'un composé activateur des aquaporines et/ou,
- au moins un composé activateur des sirtuines et/ou,
- au moins un composé qui augmente l'adhésion cellulaire et/ou,
- au moins un composé qui augmente la production de protéines matricielles telles que le collagène, fibronectine, laminine, glycosaminoglycanes et/ou,
- au moins un composé modulateur de l'activité du protéasome et/ou,
- au moins un composé modulateur du rythme circadien et/ou,
- au moins un composé modulateur des protéines HSP et/ou,
- au moins un composé qui augmente l'énergie cellulaire et/ou,
- au moins un composé modulant la pigmentation de la peau et/ou,
- au moins un composé activateur du coenzyme Q10 et/ou,
- au moins un composé améliorant la fonction barrière, tel qu'un composé activateur de la transglutaminase ou de la HMG-CoA réductase et/ou,
- au moins un composé protecteur de la mitochondrie.

Lesdits composés ci-dessus peuvent être d'origine naturelle, comme des hydrolysats peptidiques de plantes, ou encore d'origine synthétique, comme des peptides.

Indépendamment de leurs fonctions, les autres agents actifs associés à l'agent actif selon l'invention dans la composition pourront avoir des structures chimiques très diverses. On peut citer de manière non limitative, les peptides, la vitamine C et ses dérivés, les vitamines du groupe B, la DHEA (dihydroépiandrostérone), les phytostérols, l'acide salicylique et ses dérivés, les rétinoïdes, les flavonoïdes, les aminés de sucres, les azoles, les sels métalliques, les extraits peptidiques d'origine végétale ou encore les polymères.

L'invention a pour troisième objet l'utilisation d'une composition cosmétique, comprenant le peptide de formule générale (I) de séquence SEQ ID n°1-6 en tant qu'agent actif, pour augmenter la synthèse des protéines de la matrice extracellulaire par les cellules de la peau.

Plus particulièrement, le peptide selon l'invention est utilisé pour augmenter la synthèse des collagènes I et III et de la fibronectine par les fibroblastes de la peau humaine.

Selon ce mode de réalisation particulier de l'invention, le peptide de formule générale (I) de séquence SEQ ID n°1-6 est utilisé pour augmenter la densité du derme, et donc la fermeté de la peau, de retarder ou réduire le relâchement des traits du visage, la perte de volume du derme, l'amincissement de la peau, l'atonie, les ridules, les rides profondes et l'atrophie dermique.

L'invention a pour quatrième objet l'utilisation d'une composition cosmétique, comprenant le peptide de formule générale (I) de séquence SEQ ID n°1-6 en tant qu'agent actif pour augmenter l'expression de l'acide hyaluronique, à la fois par les fibroblastes et par les kératinocytes.

Selon ce mode de réalisation particulier de l'invention, le peptide de formule générale (I) de séquence SEQ ID n°1-6 est utilisé pour augmenter la capacité de toutes les couches de la peau à retenir l'eau et retarder ou réduire la déshydratation de la peau liée au vieillissement.

L'invention a pour cinquième objet l'utilisation d'une composition cosmétique, comprenant le peptide de formule générale (I) de séquence SEQ ID n°1-6 en tant qu'agent actif pour retarder l'apparition ou diminuer la sénescence des cellules de la peau.

On entend par l'expression « retarder l'apparition ou diminuer la sénescence des cellules du derme» que, selon cet aspect avantageux de l'invention, le peptide de l'invention diminue l'expression de marqueurs de sénescence dans des fibroblastes humains dont la sénescence a été induite par extinction du gène FOXO3a.

Par manifestations cutanées du vieillissement, on entend toutes modifications de l'aspect extérieur de la peau et des phanères dues au vieillissement comme, par exemple, l'amincissement de la peau, le relâchement, la perte d'hydratation et l'atonie, les rides profondes et les ridules, la perte de fermeté et de tonicité, et l'atrophie dermique ou toute autre dégradation interne de la peau consécutive à une exposition aux rayonnement UV.

D'autres avantages et caractéristiques de l'invention apparaîtront mieux à la lecture des exemples donnés à titre illustratif et non limitatif.

### Exemple 1 : Mise en évidence de l'effet activateur des peptides SEQ ID n°4 et n°5 sur l'expression du collagène I, du collagène III, du procollagène, de la fibronectine et de l'acide hyaluronique dans des fibroblastes humains

Le but de cette étude est de déterminer l'influence des peptides SEQ ID n°4 et n°5 et du peptide SEQ ID n°4 sur l'expression des protéines de la matrice extracellulaire suivantes : collagène I, collagène III, pro-collagène, fibronectine et acide hyaluronique dans les fibroblastes humains. Pour cela, des marquages spécifiques ont été réalisés sur des cultures de fibroblastes humains normaux issus du derme.

Protocole des marquages spécifiques : Des fibroblastes humains en culture sont traités avec le peptide SEQ ID n°5 ou le peptide SEQ ID n°4 à une concentration finale de 0,5%, 1% et/ou 3% (à partir d'une solution mère à 40 mg/kg), pendant 24, 48 et/ou 72 heures. Les cellules sont ensuite lavées, fixées au méthanol froid pendant 4 minutes à 4°C (pour les marquages collagène I, collagène III et acide hyaluronique) ou au formaldéhyde à 3.7% pendant 10 minutes à température ambiante (pour les marquages pro-collagène et fibronectine). Les cellules sont incubées en présence d'un anticorps polyclonal de lapin spécifique du collagène I (Rockland, Réf : 600-401-103-0.5), d'un anticorps polyclonal de lapin spécifique du collagène III (Rockland, Réf : 600-401-105-0.5), d'un anticorps polyclonal de rat spécifique du pro-collagène (Millipore, Réf : MAB1912), d'un anticorps polyclonal de lapin spécifique de la fibronectine (Sigma, Réf : F-3648), puis d'un anticorps secondaire anti-lapin couplé à un fluorochrome (Invitrogen, Réf : A21206) ou anti-rat couplé à un fluorochrome (Invitrogen, A11006). Pour le marquage de l'acide hyaluronique, les cellules sont incubées en présence d'une protéine biotinylée spécifique (Coger, Réf : 4007631A), puis en présence de streptavidine couplée à un fluorochrome (Invitrogen, Réf : S32354). Les cellules sont alors examinées au microscope à épifluorescence (Nikon Eclipse E600 microscope).

Résultats : Dans toutes les conditions testées, on observe une fluorescence plus intense dans les fibroblastes traités par le peptide SEQ ID n°5 ou le peptide SEQ ID n°4 que dans les conditions contrôle.

Conclusions : Les peptides SEQ ID n°5 et SEQ ID n°4 stimulent l'expression du collagène I, du collagène III, du pro-collagène, de la fibronectine et de l'acide hyaluronique par les fibroblastes humains dermiques.

### Exemple 2 : Mise en évidence de l'effet activateur du peptide SEQ ID n°5 sur de l'expression du collagène I, du collagène III, du pro-collagène et de l'acide hyaluronique dans des biopsies de peau

Le but de cette étude est de déterminer l'influence du peptide SEQ ID n°5 sur l'expression des protéines de la matrice extracellulaire suivantes : collagène I, collagène III, pro-collagène, fibronectine et acide hyaluronique dans la peau humaine. Pour cela des marquages spécifiques ont été réalisés sur des échantillons de peaux humaines cultivés *ex vivo.*

Protocole : Des échantillons de peau humaine sont mis en culture à l'interface air/liquide. Le peptide SEQ ID n°5 est appliqué topiquement sur les échantillons à des concentrations finales de 0,5 %, 1 % ou 3 %, (à partir d'une solution mère à 40 mg/kg) puis les échantillons sont incubés durant 24, 48 et/ou 72 heures à 37°C.

Ces échantillons de peau sont ensuite fixés avec du formaldéhyde puis inclus dans de la paraffine ou fixés et inclus dans de l'OCT puis congelés à -20° C. Des coupes de 4 µm d'épaisseur sont alors réalisées (6 µm pour les coupes à froid).

Les marquages du collagène I et du collagène III, sur les échantillons inclus en paraffine, sont effectués après démasquage des sites spécifiques. Les immunomarquages sont réalisés à l'aide d'un anticorps polyclonal de lapin spécifique du collagène I (Rockland, Ref : 600-401-103-0.5), d'un anticorps polyclonal de lapin spécifique du collagène III (Rockland, Ref: 600-401-105-0.5), puis d'un anticorps secondaire anti-lapin couplé à un fluorochrome (Invitrogen, Réf : A21206). Pour le marquage de l'acide hyaluronique, les cellules sont incubées en présence d'une protéine biotinylée spécifique (Coger, Réf : 4007631 A), puis en présence de streptavidine couplé à un fluorochrome (Invitrogen, Réf : S32354).

L'immunomarquage du pro-collagène, sur les échantillons inclus dans de l'OCT, est effectué après une fixation de 10 minutes à l'acétone froid. L'immunomarquage est réalisé à l'aide d'un anticorps polyclonal de rat spécifique du pro-collagène (Millipore, Réf : MAB1912) puis d'un anticorps secondaire anti-rat, couplé à un marqueur fluorescent (Invitrogen, A11006).

Les cellules sont alors examinées au microscope à épifluorescence (Nikon Eclipse E600 microscope).

Résultats : Les observations microscopiques montrent une fluorescence plus intense au niveau du derme des échantillons de peau traités avec le peptide SEQ ID n°5. Dans le cas de l'immunomarquage de l'acide hyaluronique, on observe également une augmentation de la fluorescence au niveau de l'épiderme des échantillons de peau traités avec le peptide SEQ ID n°5.

Conclusions : Le peptide SEQ ID n°5 stimule l'expression du collagène I, du collagène III, du pro-collagène dans le derme et stimule l'expression de l'acide hyaluronique dans le derme et l'épiderme humains.

### Exemple 3: Mise en évidence de l'effet activateur du peptide SEQ ID n°5 sur l'expression des ARNs messagers du collagène I par PCR en temps réel

Le but de cette étude est de déterminer l'influence du peptide SEQ ID n°5 sur l'expression des ARNs messagers du collagène I dans les fibroblastes humains. Pour cela, l'expression du collagène I a été étudiée par PCR en temps réel.

Protocole : Des fibroblastes humains en culture sont traités avec le peptide SEQ ID n°5 à des concentrations finales de 0,5 % et 1 % (à partir d'une solution mère à 40 mg/kg) pendant 24, 48 et 72 heures. Les ARNs totaux sont extraits à l'aide d'un kit d'extraction (QIAGEN), puis reverse transcrit avec un kit spécifique contenant des inhibiteurs de RNase (Applied Biosystems). Une PCR en temps réel est réalisée dans un thermocycleur à l'aide d'un TaqMan Gene Expression Assay spécifique du Collagène I (Applied Biosystem, Hs99999901_s1) et d'un TaqMan Gene Expression Assay spécifique du 18S utilisé comme contrôle endogène (Applied Biosystem, Hs00164004_m1). La quantification relative de l'expression des ARNs messager du Collagène I est réalisée par la méthode comparative des Ct (le Ct, threshold cycle, est l'intersection entre la courbe d'amplification et la ligne seuil).

Résultats/Conclusion : Le peptide SEQ ID n°5 stimule l'expression des ARNs messagers du Collagène I dans les fibroblastes humains.

### Exemple 4 : Etude de l'expression du marqueur de sénescence P16 en présence du peptide SEO ID n°5

Le but de cette étude est de déterminer l'influence du peptide SEQ ID n°5 sur l'expression de P16, une protéine dont l'expression dans la peau augmente avec l'âge. P16 est une protéine impliquée dans la régulation négative du cycle cellulaire, qui a été décrite comme un marqueur fiable de la sénescence cellulaire (Brookes S. et al. Expérimental Cell Research 298, 2004).

Protocole : Des fibroblastes humains en culture sont traités avec le peptide SEQ ID n°5 à des concentrations finales de 0,5 % et 1 % (à partir d'une solution mère à 40 mg/kg) pendant 48 heures. Les cellules sont préparées selon le kit Shandon Cytoblock® Cell préparation system (Thermo Scientific, Ref : 7401150).

Les cellules sont ensuite fixées au formaldéhyde puis incluses dans de la paraffine. Des coupes de 4 µm sont alors réalisées. Le marquage de p16 sur les cellules incluses en paraffine est effectué après démasquage des sites spécifiques. Les immunomarquages sont réalisés à l'aide d'un anticorps polyclonal de souris spécifique de p16 (Santa-Cruz, Ref: sc-81157), puis d'un anticorps secondaire anti-souris couplé à un fluorochrome (Invitrogen, Réf : A21202). Les cellules sont alors examinées au microscope à épifluorescence (Nikon Eclipse E600 microscope).

Résultats : Les observations microscopiques montrent une fluorescence nucléaire moins intense dans les cellules traitées par le peptide SEQ ID n°5.

Conclusions : Le peptide SEQ ID n°5 diminue l'expression du marqueur de sénescence p16 dans les fibroblastes humains.

### Exemple 5 : Mise en évidence de la réversion de la sénescence cellulaire induite chez des fibroblastes, par le peptides SEQ ID n°5

Le but de cette étude est de déterminer l'influence du peptide SEQ ID n°5 sur des fibroblastes sénescents, dont la sénescence a été induite par l'extinction du gène FOXO3a. Ces cellules surexpriment la bêta-galactosidase, en relation avec leur état sénescent. FOXO3a est un facteur de transcription de type Forkhead impliqué dans la longévité cellulaire. Dans la peau, la régulation négative de FOXO3a accélère la sénescence des fibroblastes humains normaux (Hyun Kyoung K. et al. J. of Gerontol., 2005, vol. 60A n°1, pages 4-9). Cette propriété a été mise à profit pour induire la sénescence dans des fibroblastes humains en éteignant l'expression de FOXO3a par un ARN interférant (siRNA) spécifique.

Protocole : Des fibroblastes humains en culture sont rendus sénescents par un traitement avec un siRNA spécifique de FOXO3a à une concentration finale de 25 nM en utilisant la technique de transfection par la Lipofectamine™ RNAiMAX (Invitrogen, Ref : 13778-075). Des contrôles non traités sont réalisés. Les fibroblastes sont en parallèle traités par le peptide SEQ ID n°5 à une concentration finale de 1% (à partir d'une solution mère à 40 mg/kg) pendant 48 heures.

Les cellules sont rincées et fixées dans un tampon de fixation (0,2 % glutaraldéhyde, 2 % formaldéhyde). Les cellules sont ensuite incubées à 37°C sans CO2 pendant 24 heures avec une solution de X-Gal à 1 mg/mL dans 40 mM d'acide citrique/phosphate (pH 6), 5 mM K3FeCN6, 5 mM K4FeCN6, 150 mM NaCl and 2 mM MgCl2. Les cellules sont alors examinées au microscope à lumière blanche (Nikon Eclipse E600 microscope).

Résultats : Les cellules sénescentes ayant une activité β-galactosidase spécifique sont colorées en bleu. Les cellules traitées avec le siRNA spécifique de FOXO3a montrent une augmentation de l'activité β-galactosidase liée à l'induction de la sénescence, se traduisant par une augmentation du nombre de cellules colorées en bleue. Les cellules traitées avec les siRNAs spécifiques de FOXO3a et le peptide SEQ ID n°5 à 1% montrent une diminution de l'activité β-galactosidase, se traduisant par une diminution du nombre de cellules colorées en bleu.

Conclusion : Le peptide SEQ ID n°5 est capable de diminuer le phénotype de sénescence induite dans des fibroblastes humains.

### Exemple 6 : Préparation de compositions

### 1 -Crème visage anti-âge :

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| ISP BonjourRefined shea butter | Butyrospermum Parkii ( Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Si-tec DM 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Optiphen | Phenoxyethanol (and) Caprylyl Clycol | 1 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°5 | | 3 mg/kg |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans C. A environ 45°C, le carbomer est neutralisé par addition de la phase D. A environ 30°C, la phase E est ensuite additionnée sous légère agitation et le refroidissement est poursuivi jusqu'à 25°C. La phase F est alors additionnée si souhaité.

## Revendications

1. Peptide de formule générale (I) :
R₁-(AA)ₙ-X₁-Pro-X₂-Gly-Pro-X₃-X₄-(AA)ₚ-R₂
dans laquelle
X₁ représente la glycine ou l'alanine ou la valine,
X₂ représente la glycine ou l'alanine ou la valine,
X₃ représente l'asparagine ou la lysine ou la glutamine ou aucun acide aminé,
X₄ représente la phénylalanine ou la tyrosine ou aucun acide aminé,
AA représente un acide aminé quelconque et n et p sont des nombres entiers compris entre 0 et 2,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée, soit par un groupement de type acyle (R-CO-) dans lequel le radical R est une chaîne alkyle de C₁ à C₃₀ saturée ou insaturée et de préférence un méthyle, soit par un groupement aromatique de type benzoyle, tosyle ou benzyloxycarbonyle,
R₂ représente le groupe hydroxyle de la fonction carboxyle de l'acide aminé C-terminal, -OH, dans lequel l'atome d'hydrogène peut être substitué par une chaîne alkyle de C₁ à C₃₀, ou un groupement NH₂, NHY ou NYY, dans lequel Y représente une chaîne alkyle de C₁ à C₄,
ladite séquence de formule générale (I) étant constituée de 5 à 11 résidus d'acides aminés et pouvant être sous forme de sels, **caractérisé en ce qu'**il correspond à une des séquences suivantes :
(SEQ ID n°1) : Ala-Pro-Ala-Gly-Pro-NH₂
(SEQ ID n°2) : Val-Pro-Ala-Gly-Pro
(SEQ ID n°3) : Val-Pro-Gly-Gly-Pro-NH₂
(SEQ ID n°4) : Gly-Pro-Ala-Gly-Pro
(SEQ ID n°5) : Gly-Pro-Ala-Gly-Pro-NH₂
(SEQ ID n°6) : Lys-Gly-Ala-Pro-Gly-Gly-Pro-Asn-Tyr-NH₂.

2. Peptide selon la revendication 1 **caractérisé, en ce qu'**il correspond à la séquence SEQ ID n°4 ou à la séquence SEQ ID n°5.

3. Peptide selon l'une des révendications précédentes, **caractérisé en ce qu'**il est solubilisé dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants.

4. Composition cosmétique comprenant dans un milieu physiologiquement adapté, au moins un peptide tel que défini dans l'une des revendications 1 à 3, en tant qu'agent actif.

5. Composition selon la revendication 4, **caractérisée en ce que** ledit peptide est présent à une concentration comprise entre 0,0005 à 500 mg/kg, et préférentiellement entre 0,01 à 5 mg/kg par rapport au poids total de la composition finale.

6. Composition selon l'une des revendications 4 ou 5, **caractérisée en ce qu'**elle est destinée à une administration par voie topique.

7. Composition selon l'une quelconque des revendications 4 à 6, **caractérisée en ce qu'**elle contient en outre, au moins un autre agent actif.

8. Composition selon la revendication 7, **caractérisée en ce que** l'autre agent actif est choisi parmi les agents régénérants, anti-âges, antirides, apaisants, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique ou la microcirculation ou la pousse des ongles ou la pousse des cheveux, les agents modulant la différenciation ou la pigmentation de l'épiderme, les agents inhibant les métallo-protéinases, ou encore les écrans ou filtres solaires.

9. Utilisation cosmétique d'une composition, comprenant le peptide tel que défini dans l'une des revendications 1 à 3 en tant qu'agent actif, dans un milieu physiologiquement adapté, pour augmenter la synthèse des protéines de la matrice extracellulaire par les cellules de la peau.

10. Utilisation cosmétique d'une composition, comprenant le peptide tel que défini dans l'une des revendications 1 à 3 en tant qu'agent actif, dans un milieu physiologiquement adapté, pour augmenter la synthèse des collagènes de type I et III et de la fibronectine par les fibroblastes.

11. Utilisation cosmétique d'une composition selon la revendication précédente pour augmenter la densité du derme et l'élasticité de la peau, et pour retarder ou réduire le relâchement des traits du visage, la perte de volume du derme, l'amincissement de la peau, l'atonie, les ridules, les rides profondes.

12. Utilisation cosmétique d'une composition, comprenant le peptide tel que défini dans l'une des revendications 1 à 3 en tant qu'agent actif, dans un milieu physiologiquement adapté, pour augmenter l'expression de l'acide hyaluronique par les fibroblastes et les kératinocytes de la peau.

13. Utilisation cosmétique d'une composition selon la revendication précédente pour augmenter la capacité de toutes les couches de la peau à retenir l'eau et pour retarder ou réduire la déshydratation de la peau liée au vieillissement.

14. Utilisation cosmétique d'une composition, comprenant le peptide tel que défini dans l'une des revendications 1 à 3 en tant qu'agent actif, dans un milieu physiologiquement adapté, pour retarder l'apparition ou diminuer la sénescence des cellules de la peau.

## Patentansprüche

1. Peptid der allgemeinen Formel (I):
R₁- (AA) ₙ-X₁-Pro-X₂-Gly-Pro-X₃-X₄- (AA) ₚ-R₂,
in der
X₁ für Glycin oder Alanin oder Valanin steht,
X₂ für Glycin oder Alanin oder Valanin steht,
X₃ für Asparagin oder Lysin oder Glutamin oder keine Aminosäure steht,
X₄ für Phenylalanin oder Tyrosin oder keine Aminosäure steht,
AA für eine beliebige Aminosäure steht und n und p ganze Zahlen zwischen 0 und 2 sind,
R₁ für die primäre Aminofunktion der N-terminalen Aminosäure, -NH2, steht, in der eines von zwei Wasserstoffatomen entweder durch eine gesättigte oder
ungesättigte C₁- bis C₃₀-Alkylkette, durch eine Gruppe vom Acetyl-Typ (R-CO-), in der das Radikal R eine gesättigte oder ungesättigte C₁- bis C₃₀-Alkylkette und vorzugsweise ein Methyl ist, oder durch eine aromatische Gruppe vom Benzoyl-, Tosyl- oder Benzyloxycarbonyl-Typ substituiert sein kann,
R₂ für die Hydroxylgruppe der Carboxylfunktion der C-terminalen Aminosäure, -OH, steht, in der das Wasserstoffatom durch eine C₁- bis C₃₀-Alkylkette oder eine NH₂-, NHY- oder NYY-Gruppe, in der Y für eine C₁-bis C₄-Alkylkette steht, substituiert sein kann,
wobei die besagte Sequenz der allgemeinen Formel (I) aus 5 bis 11 Aminosäureresten besteht und in der Form von Salzen sein kann, **dadurch gekennzeichnet, dass** es einer der folgenden Sequenzen entspricht:
(SEQ ID Nr. 1): Ala-Pro-Ala-Gly-Pro-NH₂
(SEQ ID Nr. 2): Val-Pro-Ala-Gly-Pro
(SEQ ID Nr. 3): Val-Pro-Gly-Gly-Pro-NH₂
(SEQ ID Nr. 4): Gly-Pro-Ala-Gly-Pro
(SEQ ID Nr. 5): Gly-Pro-Ala-Gly-Pro-NH₂
(SEQ ID Nr. 6): Lys-Gly-Ala-Pro-Gly-Gly-Pro-Asn-Tyr-NH₂.

2. Peptid nach Anspruch 1, **dadurch gekennzeichnet, dass** es der Sequenz SEQ ID Nr. 4 oder der Sequenz SEQ ID Nr. 5 entspricht.

3. Peptid nach einem der vorhergehenden Ansprüche, **dadurch gekennzeichnet, dass** es in einem oder mehreren physiologisch angepassten Lösungsmitteln, wie Wasser, Glycerin, Ethanol, Propandiol, Butylenglykol, Dipropylenglykol, ethoxylierten oder propoxylierten Diglykolen, cyclischen Polyolen oder jeglichem Gemisch dieser Lösungsmittel, solubilisiert wird.

4. Kosmetische Zusammensetzung, die in einem physiologisch angepassten Medium mindestens ein wie in einem der Ansprüche 1 bis 3 definiertes Peptid als Wirkstoff umfasst.

5. Zusammensetzung nach Anspruch 4, **dadurch gekennzeichnet, dass** das besagte Peptid in einer Konzentration zwischen 0,0005 und 500 mg/kg und vorzugsweise zwischen 0,01 und 5 mg/kg, bezogen auf das Gesamtgewicht der fertigen Zusammensetzung, vorliegt.

6. Zusammensetzung nach einem der Ansprüche 4 oder 5, **dadurch gekennzeichnet, dass** sie für eine topische Verabreichung vorgesehen ist.

7. Zusammensetzung nach einem der Ansprüche 4 bis 6, **dadurch gekennzeichnet, dass** sie außerdem mindestens einen anderen Wirkstoff enthält.

8. Zusammensetzung nach Anspruch 7, **dadurch gekennzeichnet, dass** der andere Wirkstoff aus Regenerationsmitteln, Anti-Aging-Mitteln, Antifaltenmitteln, Linderungsmitteln, Radikalfängern, Antiglykierungsmitteln, Feuchtigkeitsspendern, antibakteriellen Mitteln, Antimykotika, Keratolytika, Muskelrelaxantien, Peelingmitteln, Straffungsmitteln, Mitteln, die die Synthese von dermalen Makromolekülen oder den Energiemetabolismus oder die Mikrozirkulation oder das Nagelwachstum oder das Haarwachstum stimulieren, Mitteln, die die Differenzierung oder Pigmentierung der Epidermis modulieren, Mitteln, die die Metalloproteinasen hemmen, oder auch Sonnenschutzmitteln oder -filtern ausgewählt ist.

9. Kosmetische Verwendung einer Zusammensetzung, die das wie in einem der Ansprüche 1 bis 3 definierte Peptid als Wirkstoff in einem physiologisch angepassten Medium umfasst, zum Erhöhen der Synthese von Proteinen der extrazellulären Matrix durch die Zellen der Haut.

10. Kosmetische Verwendung einer Zusammensetzung, die das wie in einem der Ansprüche 1 bis 3 definierte Peptid als Wirkstoff in einem physiologisch angepassten Medium umfasst, zum Erhöhen der Synthese von Typ-I- und -III-Kollagenen und Fibronektin durch die Fibroblasten.

11. Kosmetische Verwendung einer Zusammensetzung nach dem vorhergehenden Anspruch zum Erhöhen der Dichte der Lederhaut und der Elastizität der Haut und zum Verzögern oder Verringern der Erschlaffung der Gesichtszüge, des Volumenverlusts der Lederhaut, des Dünnerwerdens der Haut, von Atonie, Fältchen und tiefen Falten.

12. Kosmetische Verwendung einer Zusammensetzung, die das wie in einem der Ansprüche 1 bis 3 definierte Peptid als Wirkstoff in einem physiologisch angepassten Medium umfasst, zum Erhöhen der Expression von Hyaluronsäure durch die Fibroblasten und die Keratinozyten der Haut.

13. Kosmetische Verwendung einer Zusammensetzung nach dem vorhergehenden Anspruch zum Erhöhen des Vermögens aller Schichten der Haut, Wasser zurückzuhalten, und zum Verzögern oder Verringern der mit Alterung verbundenen Dehydratisierung der Haut.

14. Kosmetische Verwendung einer Zusammensetzung, die das wie in einem der Ansprüche 1 bis 3 definierte Peptid als Wirkstoff in einem physiologisch angepassten Medium umfasst, zum Verzögern des Auftretens oder Verringern der Seneszenz von Zellen der Haut.

## Claims

1. Peptide of general formula (I):
R₁- (AA) ₙ-X₁-Pro-X₂-Gly-Pro-X₃-X₄- (AA) ₚ-R₂
wherein
X₁ represents glycine or alanine or valine,
X₂ represents glycine or alanine or valine,
X₃ represents asparagine or lysine or glutamine or no amino acid,
X₄ represents phenylalanine or tyrosine or no amino acid,
AA represents any amino acid and n and p are integers between 0 and 2,
R₁ represents the primary amine function of the N-terminal amino acid, -NH2, wherein one of the two hydrogen atoms can be substituted either by a saturated or unsaturated C₁ to C₃₀ alkyl chain, or by an acyl-type group (R-CO-) wherein the radical R is a saturated or unsaturated C₁ to C₃₀ alkyl chain and preferably a methyl, or by an aromatic group of the benzoyl, tosyl or benzyloxycarbonyl type,
R₂ represents the hydroxyl group of the carboxyl function of the C-terminal amino acid, -OH, wherein the hydrogen atom can be substituted by a C₁ to C₃₀ alkyl chain, or an NH₂, NHY or NYY group, wherein Y represents a C₁ to C₄ alkyl chain,
said sequence of general formula (I) consisting of 5 to 11 amino acid residues and capable of being in the form of salts, **characterised in that** it corresponds to one of the following sequences:
(SEQ ID n°1): Ala-Pro-Ala-Gly-Pro-NH₂
(SEQ ID n°2): Val-Pro-Ala-Gly-Pro
(SEQ ID n°3): Val-Pro-Gly-Gly-Pro-NH₂
(SEQ ID n°4): Gly-Pro-Ala-Gly-Pro
(SEQ ID n°5): Gly-Pro-Ala-Gly-Pro-NH₂
(SEQ ID n°6): Lys-Gly-Ala-Pro-Gly-Gly-Pro-Asn-Tyr-NH₂.

2. Peptide according to claim 1 **characterised in that** it corresponds to the sequence SEQ ID n°4 or the sequence SEQ ID n°5.

3. Peptide according to any of the above claims, **characterised in that** it is solubilised in one or a plurality of physiologically suitable solvents, such as water, glycerol, ethanol, propanediol, butylene glycol, dipropylene glycol, ethoxylated or propoxylated diglycols, cyclic polyols or any mixture of these solvents.

4. Cosmetic composition including, in a physiologically suitable medium, at least one peptide as defined in any of claims 1 to 3, as an active agent.

5. Composition according to claim 4, **characterised in that** said peptide is present at a concentration of between 0.0005 and 500 mg/kg, and preferably between 0.01 and 5 mg/kg with respect to the total weight of the final composition.

6. Composition according to any of claims 4 or 5, **characterised in that** it is intended for topical administration.

7. Composition according to any of claims 4 to 6, **characterised in that** it further contains at least one other active agent.

8. Composition according to claim 7, **characterised in that** the other active agent is chosen from regenerating, anti-ageing, anti-wrinkle, soothing, anti-free radical, anti-glycation, hydrating, antibacterial, antifungal, keratolytic, muscle-relaxing, exfoliating, and firming agents, agents stimulating the synthesis of dermal macromolecules or energy metabolism or microcirculation or nail or hair growth, agents modulating differentiation or pigmentation of the epidermis, metalloproteinase-inhibiting agents or sunscreens or sun filters.

9. Cosmetic use of a composition, including the peptide as defined in any of claims 1 to 3 as an active agent, in a physiologically suitable medium, for increasing extracellular matrix protein synthesis by the skin cells.

10. Cosmetic use of a composition, including the peptide as defined in any of claims 1 to 3 as an active agent, in a physiologically suitable medium, for increasing the synthesis of type-I and type-III collagens and fibronectin by fibroblasts.

11. Cosmetic use of a composition according to the above claim for increasing the density of the dermis and the elasticity of the skin, and for delaying or reducing sagging of facial features, loss of volume of the dermis, thinning of the skin, atony, fine lines and deep wrinkles.

12. Cosmetic use of a composition, including the peptide as defined in any of claims 1 to 3 as an active agent, in a physiologically suitable medium, for increasing the expression of hyaluronic acid by skin fibroblasts and keratinocytes.

13. Cosmetic use of a composition according to the above claim for increasing the capacity of all of the layers of the skin for retaining water and delaying or reducing skin dehydration related to ageing.

14. Cosmetic use of a composition, including the peptide as defined in any of claims 1 to 3 as an active agent, in a physiologically suitable medium, for delaying the appearance of or reducing senescence of skin cells.
